Europäisches Patentamt

European Patent Office

Office européen des brevets

(19)

(11) Publication number: **0 404 016**

**A1**

# EUROPEAN PATENT APPLICATION

(12)

(21) Application number: **90111464.5**

(22) Date of filing: **18.06.90**

(51) Int. Cl.5: **G01D 9/28, A61B 5/00**

(30) Priority: **23.06.89 JP 161921/89**

(43) Date of publication of application:
**27.12.90 Bulletin 90/52**

(84) Designated Contracting States:
**DE FR GB IT**

(71) Applicant: **FUKUDA DENSHI CO., LTD.**
**39-4, Hongo 3-chome Bunkyo-ku**
**Tokyo 113(JP)**

(72) Inventor: **Soejima, Ryota, c/o Fukuda Denshi**
**Co. Ltd.**
**Hongo enterprise place, 2-35-8 Hongo**
**Bunkyo-Ku, Tokyo(JP)**

(74) Representative: **Behn, Klaus, Dipl.-Ing.**
**Patentanwalt Lindenberg 34**
**D-8134 Pöcking bei München(DE)**

(54) **Method for controlling remotely recorder.**

(57) A method for controlling remotely recorder, has the following steps.

The steps consists of designating optionally data and parameterthereof at the side of a monitor, which data and parameter thereof are to be recorded in a recorder connected with the monitor.

The above data may be waveform, letter, and picture.

The parameters may be position, kind, sensitivity, and thickness in case of designating the waveform as data.

The parameters may be position, kind, and magnification in case of designating the letter as data.

The parametters may be position, longitudinal magnification, and lateral magnification in case of designating the picture as data.

EP 0 404 016 A1

## METHOD FOR CONTROLLING REMOTELY RECORDER

### BACKGROUND OF THE INVENTION

#### 1. Field of the invention

The present invention relatus to a method for controlling remotely recorder.

More particularly, it relates to a method for controlling remotely recorder, which can designate optionally, at the side of a monitor, data and parameter thereof to be recorded in a recorder.

#### 2. Description of the Related Art

Generally, a monitor is an apparatus for monitoring the condition of a patient, which monitor is connected with a recorder so as to record necessary data.

Data to be recorded in the recorder are divided broadly into three categories of waveform, letter, and picture.

The waveform is, for example, a wareform of electrocardiogram (ECG) wave form, or blood pressure wave form etc..

The letter is, for example, a Chinese character or an English character etc..

The picture is, for example, illustration or graph etc..

Regarding the above data, controlling variables , in other words, parameters are predetermined as follows.

That is to say, position, sensitivity, and thickness etc, of the waveform, a position, kind and magnification of the letter, and position, longitudinal, and lateral magnification of the picture, are predetermined respectively.

And the above data and parameters can be recorded in the recorder according to the required form.

Conventionally, since the date and parameters are fixed, they can not be designated at the side of the monitor.

Thereby, the problems with the prior art are as follows.

That is to say, with resepect to the wareform, for example, ECG wareform itsself can be recorded.

However, as a kind of ECG waveform cannot be designated optionally, various sorts of ECG waveform are not able to be recorded.

Moreover, the part of the data, for example, only the letter cannot be controlled at the side of the monitor.

As aforementioned in the prior art, recording mode is restricted regarding both data and parameters thereof, which brings about the fact that the utility value of a recorder is low.

### SUMMARY OF THE INVENTION

An object of the present invention is to elevate the utility value of a recorder, by means of designating optionally, at the side of a monitor, data and parameters thereof to be recorded in a recorder.

The above-mentioned object can be achieved by a method for controlling remotely recorder, comprising the steps of designating optionally data and parameters thereof at the side of a monitor, which data and parameter thereof are to be recorded in a recorder connected with the monitor.

### BRIEF DESCRIPTION OF THE DRAWINGS

Further features and advantages of the present invention will be apparent from the ensuring dencription with reference to the accompanying drawings, wherein:

Fig. 1 is a drawing of an embodiment in case of designating data in accordance with the present invention; and

Fig. 2A, 2B and 2C are drawings of embodiments in case of disignating parameters in accordance with the present invention.

### DESCRIPTION OF THE PREFERRED EMBODIMENTS

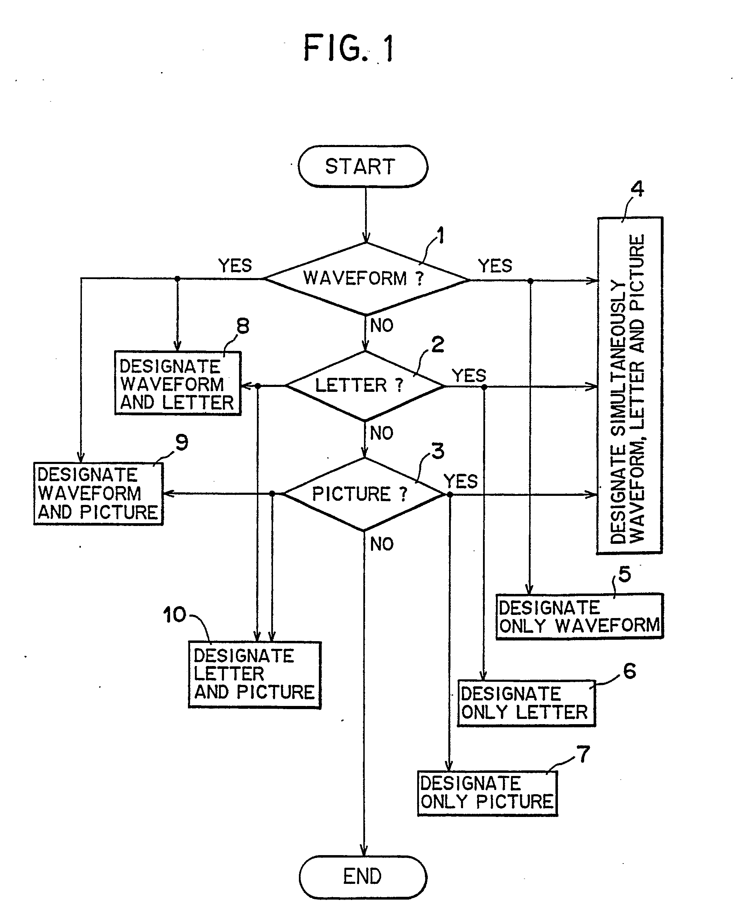

Figure 1 is a drawing of an embodiment in case of designating data in accordance with the present invention.

In this embodiment, data to be designated are 3 data of waveform, letter, and picture.

As aforementioned, the waveform is, for example, a wareform of electrocardiogram (EGG) wave form, or blood pressure waveform etc..

The letter is, for example, a Chinese character or an English character etc..

The picture is, for example, illustration or graph etc..

Now, when waveform is designated in a step 1 and data except for waveform is not designated, only waveform is designated (see step 5).

Likewise, only letter can be designated through stepes 2 and 6, and only picture can be designated through steps 3 and 7.

On the other hand, 3 data of waveform, letter, and picture can be designated simultaneously in a step 4, by designating each waveform, letter, and picture in steps 1, 2, and 3 respectively.

Moreover, 2 data of waveform and letter can be designated simultaneously in a step 8, by designating each wareform and letter in steps 1 and 2 respectively.

Likwise, 2 data of waveform and picture can be designated simultaneously in a step 9, by designating each waveform and picture in steps 1 and 3 respectively.

And, 2 data of letter and picture can be designated simultaneously in a step 10, by designating each letter and picture in steps 2 and 3 respectively.

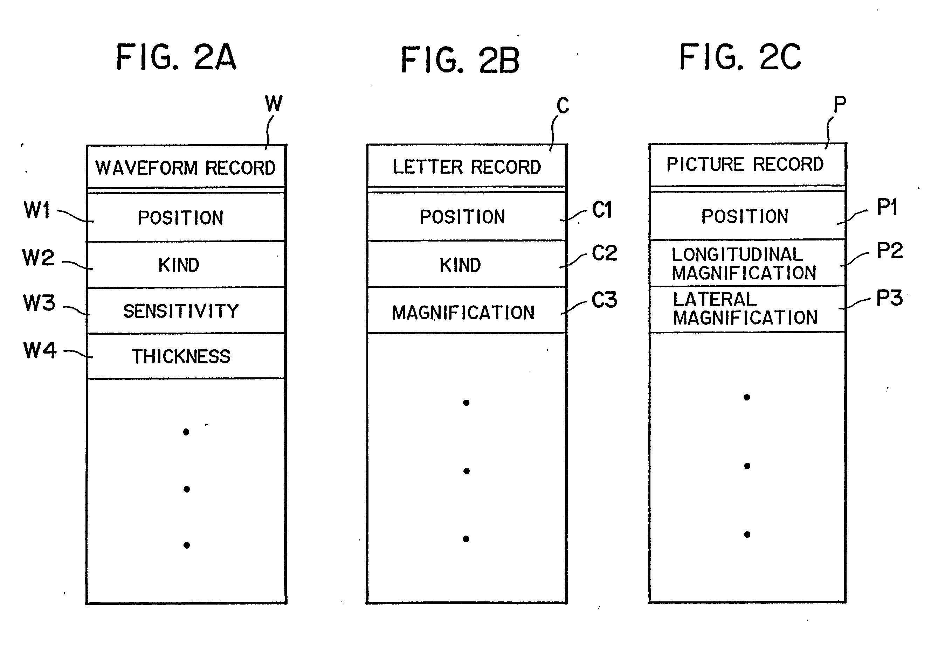

Figure 2 A to 2C are drawings of embodiments in case of designating parameters in accordance with the present invention.

Fig. 2A shows parameters in case of recording waveform W, wherein reference numeral W1 shows position, W2 kind, W3 sensitivity, W4 thickness, ● ● ●.

Meanings of the position W1 to the thickness W1 are as follows.

That is to say, the position W1 means position where waveform should be recorded on recording paper of a recorder.

The kind W2 means kind of waveform to be recorded, for example, ECG waveform or blood pressure waveform etc..

The sensitivity W3 means the degree of the size of the longitudinal direction of waveform to be recorded on recording paper.

For example, regarding ECG waveform, when the deflection of an indicator of a recorder is 10 mm against to 1 m V of ECG, the sensitivity thereof is defined as 1.

The thickness W4 means thickness of line with which waveform is drawn.

Fig. 2B shows parameters in case of recording letter C, wherein reference numeral C1 shows position, C2 kind, C3 magnification, ● ● ●.

Meanings of the position C1 to the magnification C3 are as follows.

That is to say, the position C1 means positon where character should be recorded on recording paper of a recorder.

The kind C2 means kind of character of a Chinese character or a English character etc..

The magnification C3 is the size of character to be recorded in comparison with the standard size, which is prescribed by the longitudinal and lateral directions.

Fig. 2C shows parameters in case of recording picture P, wherein reference numeral P1 shows position, P2 longitudina] magnification, C3 lateral magnification, ● ● ●.

Meanings of the position P1 to the lateral magnification P3 are as follows.

That is to say, the position P1 means position where picture should be recorded on recording paper of a recorder.

The longitudinal magnification P2 means the ratio fo a present picture to an original picture, regarding the longitudinal direction.

The lateral magnification P3 means the ratio of a present picture to an original picture, regarding the laterl direction.

The operation of the present invention will be herein after explained, in case of embodiment of recording various kinds of waveforms of ECG on recording paper of a recorder.

In this case, at first, with respect to data as shown in Fig. 1, only wareform is designated through steps 1 and 5.

Next, with respect to parameters as shown in Fig. 2A, the position W1, kind W2, sensitivity W3, and thickness W4 are designated in order.

Regarding the position W1, the upper and lowest limit positions of waveform are also included.

Regarding the kind W2, at first ECG can be designated, and next the sort of ECG 1, ECG 2 . . . thereof can be designated.

Regarding the sensitivity W3, for example, the sensitivity 1, 1/2, and 2 can be designated.

Regarding the thickness W4 thickness, with which waveform can be looked at easily, can be designated.

As aforementioned, data and parameters can be designated at the side of the monitor.

The above operation is as likwise in case of designating simultaneously 3 data through steps 1, 2, 3, and 4 as shown in Fig. 1, or in case of designating simultaneously 2 data throgh steps 1 and 8 etc. as shown in Fig 1.

For example, the operation of designating simultaneously 3 data is as follows.

That is to say, at first, waveform, letter, and pieture are designated simultaneously through steps 1, 2, 3, and 4 as shown in Fig. 1.

Next, parameters as shown in Figs. 2A to 2C are designated in order.

The position W1, kind W2, sensity W3 and thickness W4 are designated in order, regarding waveform record W as shown in Fig. 2A.

As likewise, the position C1, kind C2, and magnification C3 are designated in order, regarding letter record C as shown in Fig. 2B.

And, the position P1, longitudinal magnification P2, and lateral magnification P3 are designated in order, regarding picture record P as shown In Fig. 2C.

Moreover, the operation of designating simultaneously 2 data is as follows.

That is to say, for example, as first, waveform and letter are designated simultaneously through steps 1 and 8 as shown in Fig. 1.

Next, parameters as shown in Figs. 2A and 2B are designated in order.

The position W1, kind W2, sensitivity W3, and thickness W4 are designated In order, regarding waveform record W as shown in Fig. 2A.

As likwise, the position C1, kind C2, and wagnification C3 are designated in order, regarding letter record C as shown in Fig. 2B.

As aforementioned, according to the present invention, there is provided a method for controlling remotely recorder, comprising the steps of designating optionally data and parameter thereof at the side of a monitor, which data and parameter thereof are to be recorded in a recorder connected with the monitor.

Hence, for example, regarding data as shown in Fig. 1, all the data can be designated simultaneously through steps 1, 2, 3, and 1, any two of the data can be designated simultaneously through steps 1, 2, and 8 etc., and only any one of the data can be designated through steps 1, and 5 etc..

And, regarding parameters as shown in Fig. 2, with respect to the above designated data, for example, waveform W and letter C, positions W1 and C1 etc, where they are to be recorded on recording paper of a recorder, can be designated.

Though data and parameters therof are fixed conventionally, according to the present invention, they can be designated optionally at the side of the monitor.

Accordingly, the utility value of a recorder has become to be elevated.

## Claims

1. A method for controlling remotely recorder, comprising;
the steps of designating optionally data and parameter thereof at the side of a monitor, which data and parameter thereof are to be recorded in a recorder connected with said monitor.

2. A method for controlling remotely recorder according to claim 1,
wherein said data are waveform, letter, and picture.

3. A method for controlling remotely recorder according to claim 1,
wherein said parameters are position, kind, sensitivity, and thickness in case of designating said waveform as data,
said parameters are position, kind, and magnification in case of designating said letter as data, and
said parametters are position, longitudinal magnification, and lateral magnification in case of designating said picture as data.

# FIG. 1

## FIG. 2A

W

| WAVEFORM RECORD |
|---|
| POSITION |
| KIND |
| SENSITIVITY |
| THICKNESS |
| · · · |

W1 — POSITION
W2 — KIND
W3 — SENSITIVITY
W4 — THICKNESS

## FIG. 2B

C

| LETTER RECORD |
|---|
| POSITION |
| KIND |
| MAGNIFICATION |
| · · · |

C1 — POSITION
C2 — KIND
C3 — MAGNIFICATION

## FIG. 2C

P

| PICTURE RECORD |
|---|
| POSITION |
| LONGITUDINAL MAGNIFICATION |
| LATERAL MAGNIFICATION |
| · · · |

P1 — POSITION
P2 — LONGITUDINAL MAGNIFICATION
P3 — LATERAL MAGNIFICATION

EP 0 404 016 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 146 230 (AMERICAN HOME PRODUCTS CORP.) * Page 2, lines 11-14; page 9, line 8 - page 10, line 8 * | 1,2 | G 01 D 9/28 A 61 B 5/00 |
| A | DE-A-3 317 281 (RICOH CO., LTD) * Abstract * | 3 | |
| A | NTIS, NTN-77/0018, January 1977; "Combiner for analog and video signals" * Figure * | 3 | |
| | | | TECHNICAL FIELDS SEARCHED (Int. Cl.5) |
| | | | A 61 B 5 G 01 D |

The present search report has been drawn up for all claims

| Place of search | Date of completion of the search | Examiner |
|---|---|---|
| THE HAGUE | 01-10-1990 | LUT K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)